# EUROPEAN PATENT SPECIFICATION

(11) **EP 1 485 389 B1**
(45) Date of publication and mention of the grant of the patent: **02.08.2006**
(21) Application number: 03743815.7
(22) Date of filing: 24.02.2003
(51) Int. Cl.: C07D 493/20, A61K 31/357, A61P 33/00

(54) **ANTIPARASITIC ARTEMISININ DERIVATIVES (ENDOPEROXIDES)**
ARTEMISININDERIVATE (ENDOPEROXIDE) MIT WIRKUNG GEGEN PARASITEN
DERIVES D'ARTEMISININE ANTIPARASITAIRES (ENDOPEROXYDES)

(30) Priority: 08.03.2002 EP 02005233
(43) Date of publication of application: 15.12.2004
(73) Proprietor: Bayer HealthCare AG, 51368 Leverkusen (DE)
(72) Inventor: HAYNES, Richard K., Hong Kong Univ. Scien. & Tech., Clear Water Bay, Knowloon, Hong Kong (CN)
(86) International application number: PCT/EP2003/001839
(87) International publication number: WO 2003/076446

(56) References cited:
- WO-A-00/04024

## Description

This invention relates to certain novel C-10 substituted derivatives of artemisinin, a process for their preparation, their use in the treatment and/or prophylaxis of diseases caused by infection with a parasite and pharmaceutical compositions containing such C-10 substituted derivatives.

Malaria is the most important human parasitic disease in the world today. Approximately 270 million people throughout the world are infected with malaria, with about 2 million dying each year. The ability of parasites to produce a complex survival mechanism by expressing variant antigens on the surface of infected erythrocytes makes it possible for the parasites to escape from the destructive action of the host immune response against these antigens. In addition, the increasing rate of malaria infection is due to the spread of chloroquine-resistant strains of Plasmodium falciparum and the other multi-drug resistant strains.

In the field of animal health, parasitic diseases are a major problem, especially those diseases which are functionally related to malaria. For instance, neosporosis is a term used to describe diseases caused by parasites of the species Neospora, especially Neospora caninum, in animals. Neospora infections are known to occur in dogs, cattle, sheep, goats and horses.

The final host for Neospora spp., including Neospora caninum, is unknown and, in addition, the complete cycle of development of the parasite is not understood. The asexual phases of reproduction, known as schizogony, and the behaviour of the unicellular tachyzoite/bradyzoite stage have been clarified, however. Tachyzoites are infectious unicellular parasite stages of about 3-7 x 1-5 mm in size formed after intracellular reproduction termed endodyogeny. Reproduction via tachyzoites takes place preferentially in organelles such as muscle or nerve cells. Pathological symptoms invoked after an infection are associated mainly in those tissues. Some five to six weeks after natural infection in a dog, symptoms of the disease are hypersensitivity caused by inflammation of neuronal cells and increasing tendency to hyperextension of the hind legs. Histopathological lesions are apparent in the nervous system, preferentially in the brain and spinal cord. Extensive non-suppurative inflammations, glial excrescences and perivascular infiltrations of mononuclear cells (macrophages, lymphocytes, plasma cells) dominate, and are also partly apparent in eosinophils and neutrophils. In the muscular system, macroscopically observable necroses and degenerative changes appear. Apart from the more or less strongly developed atrophy, long pale longitudinal stripes are evident.

In California and Australia, Neospora caninum infections appear to be the main cause for abortion in cattle. Symptoms of the disease in cattle are similar to those in the dog. Ataxia is apparent, joint reflexes are weakened and pareses at the hind legs, partly in all four legs, can be observed. The histological picture is similar to that of the dog; mainly non-suppurative meningitis and myelitis.

Data on in vivo activity of compounds suitable against neosporosis are rare because adequate in vivo test systems still have to be developed. Sulfadiazin (administered via drinking water) is effective in experimentally infected mice, only if the treatment was prophylactic, that is, the treatment was started before infection. In dogs, treatment with sulfadiazin and clindamycin is only successful if it is started early, that is, at the appearance of first clinical symptoms as a result of neuronal inflammation.

Coccidiosis, an infection of the small intestine, is relatively rarely diagnosed in humans, where it is caused by Isospora belli. However, humans are also the final host of at least two cyst-forming coccidial species (Sarcocystis suihominis and S. bovihominis). Consumption of raw or inadequately cooked pork or beef containing such cysts can lead to severe diarrhoea, the cause of which is probably seldom diagnosed correctly. Coccidia (phylum Apicomplexa, suborder Eimeriina) are one of the most successful groups of parasitic protozoans, having conquered virtually every class of Metazoa. The ones that are of particular importance for man are the 60-100 species which parasitise domestic animals and which in some instances can cause very severe losses, especially in poultry, although also in lambs, calves, piglets, rabbits and other animals (see Table A).

**Table A: Causatives of intestinal coccidiosis in domestic animals**

| Animal | number of Eimeria and/or Isospora species*) | most pathogenic and/or very common species (E=Eimeria, I=Isospora) |
|---|---|---|
| chicken (Gallus gallus) | 7 | E.tenella, E.necatrix, E.maxima, E.acerviilina |
| turkey (Meleargidis gallopavo) | 7 | E.meleagrimitis, E.adenoides |
| goose (Anser anser) | 6 | E.anseris, E.truncata, E.nocens, E. kotlani |
| duck (Anas platyhynehus) | 3 | Tyzzeria perniciosa, E.anatis |
| pigeon (Columba livia) | 2 | E.columbarum, E.labbeanea |
| rabbit (Oryctolagus cuniculus) | 11(12) | E.intestinalis, E.flavescens; E.stiedai, E.magna, E.perforans |
| sheep (Ovis arius) | 11(16) | E.ovinoidalis, E.ashata, E.ovina |
| goat (Capra hircus) | 12(15) | E.ninakohlyakimovae,E.arloingi |
| cattle (Bos taurus) | 12(15) | E.zuernii, E.bovis, E.auburnensis |
| pig Sus scofra) | 7(14) | I.suis, E.debliecki, E.scabra |
| dog (Canis familiaris) | 5 | I.canis, I.(Cystisospora) burrowsi |
| cat (Felis catus) | 2+6 | I.felis, I.rivolta as final host: Sarcocystis bovifelis, S.ovifelis, S.fusiformis, S.muris, S.cuniculi, Toxoplasma gondii |

| | | |
|---|---|---|
| *) regarding to Pellerdy (1974), Eckert et al, (1995b, Levine and Ivens (1970) and Mehlhorn 1988) | | |

Most of the pathogenic species are strictly host-specific. They have a complex life cycle with two asexual reproduction phases (schizogony or merogony, and sporogony) and a sexual development phase (gametogony). In view of the major importance of coccidiosis, numerous reviews are available, for instance, by Davies et al. (1963), Hammond and Long (1973), Long (1982, 1990), and Pellerdy (1974). The econonucally important species sometimes differ very considerably in their sensitivity to medicinal active ingredients. The sensitivity of the different developmental stages to medicinal agents also varies enormously.

As far as the use of drugs is concerned, prophylaxis is the main approach in poultry, in which symptoms do not appear until the phase of increased morbidity, and therapy is the principal strategy in mammals (McDougald 1982). Polyether antibiotics and sulfonamides, among other drugs, are currently used for such treatment and prophylaxis. However, drug-resistant strains of Eimeria have emerged and drug-resistance is now a serious problem. New drugs are therefore urgently required. Given the multiplicity of pathogens and hosts, there is no "ideal model" for identifying and testing anticoccidial agents. For example, most of the many substances used for preventing coccidiosis in poultry are insufficiently effective or even completely ineffective against mammalian coccidia (Haberkorn and Mundt; 1989; Haberkom 1996). Numerous works and sets of instructions have been published on testing of active ingredients in animals for anticoccidial efficacy, for immunisation, etc. One particularly important and comprehensive example is the survey of current methods published by Eckert et al. (1995a).

The compound artemisinin, also known as qinghaosu (1), is a tetracyclic 1,2,4-trioxane occurring in Artemisia annua. Artemisinin and its derivatives dihydroartemisinin (2), artemether (3) and sodium artesunate (4) have been used for the treatment of malaria.

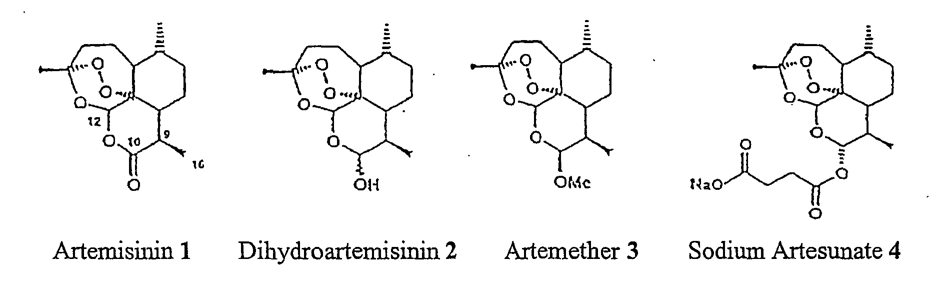

Different modes of action have been proposed by various groups to account for the action of artemisinin and its derivatives in treating malaria (Posner et al., *J. Am. Chem. Soc. **1996**, 118, 3537;* Posner et al., *J. Am. Chem. Soc. **1995**, 117, 5885;* Posner et al., *J. Med. Chem. **1995**, 38, 2273).* However, irrespective of actual mode of action, all current derivatives suffer from poor oral bioavailability and poor stability (Meshnick et al., *Parasitology Today **1996**, 12, 79),* especially the 'first generation' ethers and esters artemether and sodium artesunate obtained from dihydroartemisinin. Extensive chemical studies carried out on artemisinin and derivatives indicate that a cause of instability is the facile opening of the trioxane moiety in artemisinin itself, or in the metabolite common to all currently used derivatives artemether, arteether and artesunate, namely dihydroartemisinin. Ring opening will provide the free hydroperoxide, which is susceptible to reduction. Removal of this group ensures destruction of drug activity with the reduction products being transformed into desoxo metabolites. In order to render ring-opening less facile, the oxygen atom at C-10 can be either removed to provide 10-deoxydihydroartemisinin, or replaced by other groups, and this has provided the basis for the so-called 'second generation' compounds which are generally 10-deoxy artemisinin derivatives. In addition, derivatives of artemisinin have also been prepared with a variety of substituents at C-9.

Artemisinin derivatives are also known in which the oxygen atom at C-10 has been replaced by an amine group. For instance, Yang et al (Biorg. Med. Chem. Lett., **1995**, 5, 1791-1794) synthesised ten new artemisinin derivatives in which the oxygen atom at C-10 was replaced by a group -NHAr where Ar represents a phenyl, 3-chlorophenyl, 4-chlorophenyl, 3-bromophenyl, 4-bromophenyl, 4-iodophenyl, 4-methylphenyl, 4-methoxyphenyl, 3-carboxylphenyl or 4-carboxylphenyl group. These compounds were tested for in vivo activity against the K173 strain of Plasmodium berghei and found to be active.

WO 00/04024 discloses further C-10 substituted derivatives of artemisinin.

Whilst the current artemisinin derivatives are successful, there are problems associated with stability, bioavailability and potential neurotoxicity. There is also a need for artemisinin derivatives which exhibit a broad spectrum of activity against a variety of parasites.

It has now been discovered that certain C-10 substituted derivatives of artemisinin are effective in the treatment of diseases caused by infection with a parasite. These compounds are particularly effective in the treatment of diseases caused by infection with a parasite of the genera Plasmodium, Neospora or Eimeria, especially Plasmodium falciparum, Neospora caninum and Eimeria tenella which cause malaria, neosporosis and coccidiosis respectively.

According to the present invention there is therefore provided a compound of the general formula I 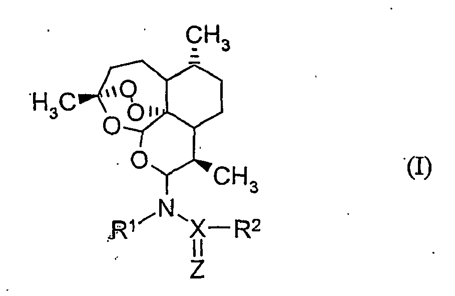 or a salt thereof, or a solvate thereof, or a solvate of a salt thereof,
in which
- R¹: represents a hydrogen atom or an optionally substituted alkyl, alkenyl, alkynyl, cycloalkyl, aryl or aralkyl group;
- X: represents a carbon atom, a sulfur atom, a sulfoxide group S=O or a group PR³, P-O-R³ or P-N(R⁴)-R³ where R³ and R⁴ each independently represent a hydrogen atom or an optionally substituted alkyl, alkenyl, alkynyl, cycloalkyl, aryl or aralkyl group;
- Z: represents an oxygen atom, a sulfur atom or a group NR⁵ where R⁵ represents a hydrogen atom or an optionally substituted alkyl, alkenyl, alkynyl, cycloalkyl, aryl or aralkyl group; and
- R²: represents a hydrogen atom or an optionally substituted alkyl, alkenyl, alkynyl, cycloalkyl, aryl or aralkyl group, or a group N(R⁶)₂, NHNH₂, NR⁶NHR⁶ or NR⁶N(R⁶)₂, or a group OR⁶ or SR⁶ where each R⁶ independently represents a hydrogen atom or an optionally substituted alkyl, alkenyl, alkynyl, cycloalkyl, aryl or aralkyl group, or a 10α-dihydroartemisinyl group, or R² represents a group OR⁷ or NR⁶R⁷ where R⁶ represents a group as defined above and R⁷ represents a bond attached as a substituent to R⁵ together with the interjacent group -X=Z- forming an optionally substituted heterocyclic group where Z represents a group NR⁵, or R⁷ represents a bond attached as a substituent to R¹ together with the interjacent group -N-X(=Z)- forming an optionally substituted heterocyclic group.

Suitable salts include acid addition salts and these may be formed by reaction of a suitable compound of formula I with a suitable acid, such as an organic acid or a mineral acid. Acid addition salts formed by reaction with a mineral acid are particularly preferred, especially salts formed by reaction with hydrochloric or hydrobronlic acid.

A solvate according to the present invention is any such form of the compounds forming a complex in solid or liquid state by coordination with solvent molecules. Hydrates are a special form of solvates formed by coordination with water molecules.

Any alkyl, alkenyl or alkynyl group, unless otherwise specified, may be linear or branched and may contain up to 12, preferably up to 6, and especially up to 4 carbon atoms. Preferred alkyl groups are methyl, ethyl, propyl and butyl. It is preferred that any alkenyl or alkynyl group is not an alk-1-enyl or alk-1-ynyl group. In other words, there should preferably be at least one methylene group -CH₂- or similar sp³-hybridised center between a carbon atom forming part of the double or triple C-C bond and the nitrogen atom to which the group is attached. Preferred alkenyl and alkynyl groups include propenyl, butenyl, propynyl and butynyl groups. When an alkyl moiety forms part of another group, for example the alkyl moiety of an aralkyl group, it is preferred that it contains up to 6, especially up to 4, carbon atoms. Preferred alkyl moieties are methyl and ethyl.

An aryl group may be any aromatic hydrocarbon group and may contain from 6 to 24, preferably 6 to 18, more preferably 6 to 16, and especially 6 to 14, carbon atoms. Preferred aryl groups include phenyl, naphthyl, anthryl, phenanthryl and pyryl groups, especially a phenyl or naphthyl, and particularly a phenyl, group. When an aryl moiety forms part of another group, for example the aryl moiety of an aralkyl group, it is preferred that it is a phenyl, naphthyl, anthryl, phenanthryl or pyryl, especially phenyl or naphthyl, and particularly a phenyl, moiety.

An aralkyl group may be any alkyl group substituted by an aryl group. A preferred aralkyl group contains from 7 to 30, particularly 7 to 24 and especially 7 to 18, carbon atoms, particularly preferred aralkyl groups being benzyl, naphthylmethyl, anthrylmethyl, phenanthrylmethyl and pyrylmethyl groups. A particularly preferred aralkyl group is a benzyl group.

A cycloalkyl group may be any saturated cyclic hydrocarbon group and may contain from 3 to 12, preferably 3 to 8, and especially 3. to 6, carbon atoms. Preferred cycloalkyl groups are cyclopropyl, cyclopentyl and cyclohexyl groups.

A heteroaryl group may be any aromatic monocyclic or polycyclic ring system which contains at least one heteroatom. Preferably, a heteroaryl group is a 5- to 18-membered, particularly a 5- to 14-membered, and especially a 5- to 10-membered, aromatic ring system containing at least one heteroatom selected from oxygen, sulphur and nitrogen atoms. Preferred heteroaryl groups include pyridyl, pyrylium, thiopyrylium, pyrrolyl, furyl, thienyl, indolinyl, isoindolinyl, indolizinyl, imidazolyl, pyridonyl, pyronyl, pyrimidinyl, pyrazinyl, oxazolyl, thiazolyl, purinyl, quinolinyl, isoquinolinyl, quinoxalinyl, pyridazinyl, benzofuranyl, benzoxazolyl and acridinyl groups. A C-linked heteroaryl group is therefore a heteroaryl group as defined above which is linked to the tetracyclic 1,2,4-trioxane moiety of a compound of general formula I via a carbon atom in the heteroaromatic ring system.

A heterocyclic group may be any monocyclic or polycyclic ring system which contains at least one heteroatom and may be unsaturated or partially or fully saturated. The term "heterocyclic" thus includes heteroaryl groups as defined above as well as non-aromatic heterocyclic groups. Preferably, a heterocyclic group is a 3-to 18- membered, particularly a 3- to 14-membered, especially a 5- to 10-membered, ring system containing at least one heteroatom selected from oxygen, sulphur and nitrogen atoms. Preferred heterocyclic groups include the specific heteroaryl groups named above as well as pyranyl, piperidinyl, pyrrolidinyl, dioxanyl, piperazinyl, morpholinyl, thiomorpholinyl, morpholinosulphonyl, tetrahydroisoquinolinyl and tetrahydrofuranyl groups.

A heterocyclylalkyl group may be any alkyl group substituted by a heterocyclic group. Preferably, the heterocyclic moiety is a 3- to 18-membered, particularly a 3-to 14-membered, and especially a 5- to 10-membered, heterocyclic group as defined above and the alkyl moiety is a C₁₋₆ alkyl, preferably C₁₋₄ alkyl, and especially methyl, group.

An amino acid may be any α-amino acid, such as glycine, alanine, valine, leucine, isoleucine, serine, threonine, cysteine, cystine, methionine, aspartic acid, glutamic acid, aspargine, glutamine, lysine, hydroxylysine, arginine, histidine, phenylalanine, tyrosine, tryptophan, proline, hydroxyproline or phenylglycine, and includes both D-and L-configurations. An amino acid ester may be any ester of such an amino acid, alkyl esters, particularly C₁₋₄ alkyl esters, being especially preferred.

When any of the foregoing substituents are designated as being optionally substituted, the substituent groups which are optionally present may be any one or more of those customarily employed in the development of pharmaceutical compounds and/or the modification of such compounds to influence their structure/activity, stability, bioavailability or other property. Specific examples of such substituents include, for example, halogen atoms, nitro, cyano, hydroxyl, cycloalkyl, alkyl, alkenyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, alkylsulphonato, arylsulphinyl, arylsulphonyl, arylsulphonato, carbamoyl, alkylamido, aryl, heterocyclic and alkyl- or aryl-substituted heterocyclic groups. When any of the foregoing substituents represents or contains an alkyl or alkenyl substituent group, this may be linear or branched and may contain up to 12, preferably up to 6, and especially up to 4, carbon atoms. A cycloalkyl group may contain from 3 to 8, preferably from 3 to 6, carbon atoms. An aryl group or moiety may contain from 6 to 10 carbon atoms, phenyl groups being especially preferred. A heterocyclic group or moiety may be a 5- to 10-membered ring system as defined above. A halogen atom may be a fluorine, chlorine, bromine or iodine atom and any group which contains a halo moiety, such as a haloalkyl group, may thus contain any one or more of these halogen atoms.

In one aspect, it is preferred, that R¹ represents a hydrogen atom, a methyl group, ethyl group or longer chain alkyl group or a branched alkyl group containing up to 9 carbon atoms, preferably a hydrogen atom, a methyl group or an ethyl group.

In another preferred aspect, X represents a carbon atom, a sulfur atom, or a group PR³, P-O-R³ or P-N(R⁴)-R³ where R³ and R⁴ each independently represent a C₆₋₁₈ aryl group or a 5- to 10-membered C-linked heteroaryl group or a 5- to 10-membered heterocyclyl-C₁₋₆ alkyl group optionally substituted by one or more substituents selected from the group consisting of halogen atoms, hydroxyl, C₁₋₄ alkyl, C₂₋₄. alkenyl, C₁₋₄ haloalkyl, C₁₋₄ alkoxy, C₁₋₄ haloalkoxy, amino, C₁₋₄ alkylamino, di(C₁₋₄ alkyl)amino and carboxyl groups. Preferably, X represents a carbon atom or a sulfur atom.

In a further preferred aspect, Z represents an oxygen atom, or a group NR⁵ where R⁵ represents a hydrogen atom, a methyl group, ethyl group or longer chain alkyl group or branched alkyl group containing up to 9 carbon atoms, or a C₆₋₁₈ aryl group or a 5-to 10-membered C-linked heteroaryl group or a 5- to 10-membered heterocyclyl-C₁₋₆ alkyl group optionally substituted by one or more substituents selected from the group consisting of halogen atoms, hydroxyl, C₁₋₄ alkyl, C₂₋₄ alkenyl, C₁₋₄ haloalkyl, C₁₋₄ alkoxy, C₁₋₄ haloalkoxy, amino, C₁₋₄ alkylamino, di(C₁₋₄ alkyl)amino and carboxyl groups.

In another preferred aspect, R² represents a hydrogen atom or an optionally substituted alkyl, alkenyl, alkynyl, cycloalkyl, aryl or aralkyl group, or a group OR⁶, SR⁶, NH₂, NHR⁶, or N(R⁶)₂ where each R⁶ indepently represents a methyl group, ethyl group or longer chain alkyl group or branched alkyl group containing up to 9 carbon atoms atoms, or is a C₆₋₁₈ aryl group or a 5- to 10-membered C-linked heteroaryl group or a 5- to 10-membered heterocyclyl-C₁₋₆ alkyl group optionally substituted by one or more substituents selected from the group consisting of halogen atoms, hydroxyl, C₁₋₄ alkyl, C₂₋₄ alkenyl, C₁₋₄ haloalkyl, C₁₋₄ alkoxy, C₁₋₄ haloalkoxy, amino, C₁₋₄ alkylamino, di(C₁₋₄ alkyl)amino and carboxyl groups. Preferably, R² represents a group NH₂, or a group NHR⁶ where R⁶ represents an alkyl or aryl group, or a group N(R⁶)₂ where R⁶ represent identical or differentiated alkyl groups.

In a further preferred aspect, R¹ represents a hydrogen atom or an optionally substituted alkyl, alkenyl, alkynyl, cycloalkyl, aryl or aralkyl group, preferably a hydrogen atom or an alkyl group, more preferably a hydrogen atom or a methyl group or an ethyl group; X represents a carbon, phosphorus or sulfur atom, preferably a carbon or sulfur atom; Z represents an oxygen atom or a group NR⁵ in where R⁵ represents a hydrogen atom or an optionally substituted alkyl, alkenyl, alkynyl, cycloalkyl, aryl or aralkyl group, preferably an oxygen atom; and R² represents a group OR⁶, SR⁶, NH₂, NHR⁶, or N(R⁶)₂ where each R⁶ independently represents a hydrogen atom or an optionally substituted alkyl, alkenyl, alkynyl, cycloalkyl, aryl or aralkyl group, or a 10α-dihydroartemisinyl group, preferably a hydrogen atom or an optionally substituted alkyl or aryl group, more preferably R² represents a group NH₂, or a group NHR⁶ where R⁶ represents an alkyl group, or a group N(R⁶)₂ where R⁶ represent identical or differentiated alkyl groups.

In an especially preferred aspect, R¹ represents a hydrogen atom, X represents a sulfoxide group S=O, Z represents an oxygen atom, and R² represents a group NH₂; or R¹ represents a hydrogen atom, X represents a carbon atom, Z represents a group NH, and R² represents a group NHR⁶ where R⁶ represents a hydrogen atom or an optionally substituted alkyl, cycloalkyl, aryl or aralkyl group; or R¹ represents a hydrogen atom, X represents a carbon atom, Z represents an oxygen atom, and R² represents a group NHR⁶ where R⁶ is a hydrogen atom or an optionally substituted alkyl, cycloalkyl, aryl or aralkyl group.

It should also be appreciated that the compounds of general formula I are capable of existing as different geometric and optical isomers. The present invention thus includes both the individual isomers and mixtures of such isomers.

The present invention includes a compound of the general formula I as defined above for use in the treatment and/or prophylaxis of a disease. Preferably, the disease is a disease caused by infection with a parasite. More preferably, the disease is a disease caused by infection with a parasite of the genus Plasmodium, the genus Neospora, or the genus Eimeria.

The present invention also provides the use of a compound of the general formula I as defined above for the manufacture of a medicament for the treatment and/or prophylaxis of a disease caused by infection with a parasite. Preferably, the parasite is an organism of the genus Plasmodium, the genus Neospora, or the genus Eimeria.

The present invention also provides a process for the preparation of a compound of the general formula I which comprises reacting a compound of the general formula II 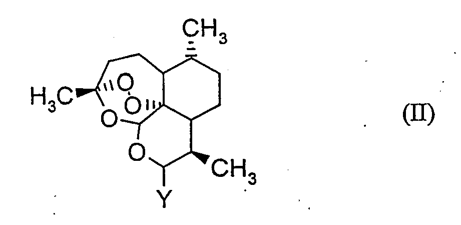 in which Y represents a group containing an oxygen atom attached to the carbon atom of the artemisinin nucleus and also to a hydrogen atom or trimethylsilyl group, with a suitable halogenating agent to form a compound of the general formula II in which Y represents a halogen atom; and, if desired, reacting the compound of general formula II thus formed with an amine of the general formula R¹NHX(=Z)R² where R¹, R², X and Z are as defined above to form a compound of general formula I.

Suitable halogenating agents for forming compounds of the general formula II in which Y represents a halogen atom include diethylaminosulphur trifluoride, chlorotrimethylsilane, bromotrimethylsilane and iodotrimethylsilane. In particular, compounds of the general formula II in which Y represents a chlorine, bromine or iodine atom may be prepared by reacting a compound of the general formula II in which Y represents a trimethylsilyloxy group with a suitable chlorinating, brominating or iodinating agent respectively, such as chlofotrimethlysilane, bromotrimethylsilane or iodotrimethylsilane respectively. This reaction may be conveniently carried out in the presence of a solvent. Suitable solvents include aromatic solvents such as toluene, or halogenated hydrocarbons, especially chlorinated hydrocarbons, such as dichloromethane. Preferably, the reaction is carried out at a temperature of -30 to +20°C, particularly -5 to +10°C, about 0°C being especially preferred.

Compounds of the general formula II in which Y represents a fluorine atom may be conveniently prepared by reacting a compound of the general formula II in which Y represents a hydroxyl group with a suitable fluorinating agent, such as diethylaminosulphur trifluoride. This reaction may be conveniently carried out in the presence of a solvent, suitable solvents including aromatic solvents such as toluene and halogenated hydrocarbons, especially chlorinated hydrocarbons, such as dichloromethane. Preferably, the reaction is carried out at -5°C to room temperature, that is, -5 to +35°C, preferably 0 to 30°C. The reaction may also be carried out under an inert atmosphere, such as nitrogen.

The reaction of an amine of the type R¹NHX(=Z)R² where R¹, R², X and Z are as defined above with a compound of the general formula II in which Y represents a halogen, preferably a chlorine or bromine, atom to form a compound of the general formula II in which Y represents the group R¹NX(=Z)R² where R¹, R², X and Z are as defined above may be conveniently carried out in the presence of a solvent. Suitable solvents include halogenated hydrocarbons, especially chlorinated hydrocarbons, such as dichloromethane, and ethers, such as tetrahydrofuran. Preferably, the reaction is carried out at a temperature of -5 to +5°C, 0°C being especially preferred.

When a compound of the general formula II in which Y represents a bromine atom is to be further reacted with an amine to form a compound of the general formula II in which Y represents a group R¹NHX(=Z)R² where R¹, R², X and Z are as defined above, it is preferred that the compound of the general formula II in which Y represents a bromine atom is generated in situ by reacting a compound of the general formula II in which Y represents a trimethylsiloxy group with bromotrimethylsilane.

A compound of the general formula II in which Y represents a trimethylsiloxy group may be prepared by reacting dihydroartemisinin, that is, the compound of general formula II in which Y represents a hydroxyl group, with chlorotrimethylsilane in the presence of a base, such as pyridine or triethylamine. Preferably, the reaction is carried out at room temperature, that is, 15 to 35°C, preferably 20 to 30°C.

Dihydroartemisinin, that is, the compound of general formula II in which Y represents a hydroxyl group, is a known compound and can be prepared by known processes.

The present invention also provides a pharmaceutical composition which comprises a carrier and, as active ingredient, a compound of the general formula I as defined above.

A pharmaceutically acceptable carrier may be any material with which the active ingredient is formulated to facilitate administration. A carrier may be a solid or a liquid, including a material which is normally gaseous but which has been compressed to form a liquid, and any of the carriers normally used in formulating pharmaceutical compositions may be used. Preferably, compositions according to the present invention contain 0.5 to 95% by weight of active ingredient.

The compounds of general formula I can be formulated as, for example, tablets, capsules, suppositories or solutions. These formulations can be produced by known methods using conventional solid carriers such as, for example, lactose, starch or talcum or liquid carriers such as, for example, water, fatty oils or liquid paraffins. Other carriers which may be used include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; polypeptide/protein or polysaccharide complexes such as gelatin-acacia complexes; sugars such as mannitol, dextrose, galactose and trehalose; cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

Auxiliary components such as tablet disintegrants, solubilisers, preservatives, antioxidants, surfactants, viscosity enhancers, colouring agents, flavouring agents, pH modifiers, sweeteners or taste-masking agents may also be incorporated into the composition. Suitable colouring agents include red, black and yellow iron oxides and FD & C dyes such as FD & C blue No. 2 and FD & C red No. 40 available from Ellis & Everard. Suitable flavouring agents include mint, raspberry, liquorice, orange, lemon, grapefruit, caramel, vanilla, cherry and grape flavours and combinations of these. Suitable pH modifiers include citric acid, tartaric acid, phosphoric acid, hydrochloric acid and maleic acid. Suitable sweeteners include aspartame, acesulfame K and thaumatin. Suitable taste-masking agents include sodium bicarbonate, ion-exchange resins, cyclodextrin inclusion compounds, adsorbates or microencapsulated actives.

For treatment of and prophylaxis against coccidiosis and related parasites, for instance, in poultry, especially in chickens, ducks, geese and turkeys, 0.1 to 100 ppm, preferably 0.5 to 100 ppm of the active compound may be mixed into an appropriate, edible material, such as nutritious food. If desired, the amounts applied can be increased, especially if the active compound is well tolerated by the recipient. Accordingly, the active compound can be applied with the drinking water.

For the treatment of a single animal, for instance, for the treatment of coccidiosis in mammals or toxoplasmosis, amounts of 0.5 to 100 mg/kg body weight active compound are preferably administered daily to obtain the desired results. Nevertheless, it may be necessary from time to time to depart from the amounts mentioned above, depending on the body weight of the experimental animal, the method of application, the animal species and its individual reaction to the drug or the kind of formulation or the time or interval in which the drug is applied. In special cases, it may be sufficient to use less than the minimum amount given above, whilst in other cases the maximum dose may have to be exceeded. For a larger dose, it may be advisable to divide the dose into several smaller single doses.

The present invention also includes a pharmaceutical composition as described above for use in the treatment and/or prophylaxis of a disease caused by infection with a parasite. Preferably, the parasite is an organism of the genus Plasmodium, the genus Neospora, or the genus Eimeria.

The present invention is further illustrated by the following examples.

### Examples

### Example 1: 10α-(Sulfamino)dihydroartemisinin

### (Formula I: R¹ = H; X = S=O; Z = O; R² = NH₂)

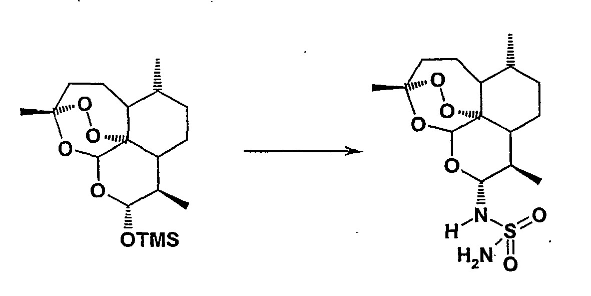

Trimethylsilyl bromide (0.16 g, 0.14 ml, 1.05 mmol) was added dropwise to a cold (0 °C) stirred solution of 10a-(trimethylsiloxy)dihydroartemisinin (356 mg, 1.0 mmol) in dichloromethane (5 ml). After 15 min. (tlc), a solution of sulfamide (0.19 g, 2.0 mmol) in THF (6 ml) was added. After 1.5h, the reaction was quenched with saturated NaHCO_{3(aq)} (10 ml) and extracted with diethyl ether (3 x 10 ml). The organic extracts were combined and dried (MgSO₄). Filtration and evaporation of filtrate gave a dark green solid which was purified by column chromatography on silica with ethyl acetate-hexanes (40:60) as eluent. Pooling and evaporation of appropriate fractions gave a white powder (211.48 mg, 57%). M.p. 168-168.7 °C (decomposed); [α]_{D}²² +16.76° (c 0.68 MeOH); IR (KBr) νₘₐₓ 3387, 3226, 2959, 2934, 2880, 1631, 1456, 1375, 1323, 1308, 1147, 1128, 1024; ¹H-NMR: δ_{H} 7.79 (1H, d, NH, *J* = 8.63 Hz), 6.44 (2H, s, NH₂), 5.37 (1H, s, H-12), 4.58 (1H, pseudo-triplet, H-10, *J*= 9.23 Hz), 2.31-2.12 (2H, m), 2.00-1.96 (1H, m), 1.82-1.77 (1H, m), 1.64-1.61 (2H, m), 1.51-1.31 (4H, m), 1.28 (3H, s, 3-Me), 1.20-1.12 (1H, m), 1.10-0.94 (1H, m), 0.89 (3H, d, 9-Me, *J*= 6.23 Hz), 0.78 (3H, d, 6-Me, *J*= 7.11 Hz); ¹³C-NMR: δ_{c} 103.31, 90.61, 80.47, 79.98, 51.36, 45.14, 36.34, 36.01, 33.66, 31.56, 25.66, 24.57, 21.24, 20.46, 13.60; MS (CI, CH₄) *m*/*z* 363 (MH⁺, 7%), 364 (MH⁺, ¹³C, 1%); Analysis calculated for C₁₅H₂₆N₂O₆S requires C, 49.71; H, 7.23; N, 7.73; found C, 49.59; H, 7.29; N, 7.58.

### Example 2: Bis[(10α-dihydroartemisinyl)]sulfamide

### (Formula I: R¹ = H; X = S=O; Z = O; R² = NH(10α-dihydroartemisinyl))

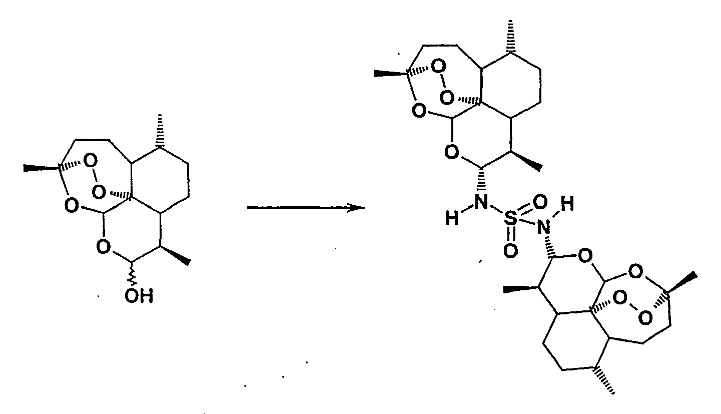

Trimethylsilyl chloride (0.42 g, 0.49 ml, 3.87 mmol) was added to a cold (0 °C) stirred mixture of dihydroartemisinin (0.5 g, 1.76 mmol) and sodium bromide (199 mg, 1.94 mmol) in toluene (2 ml). After 1h (t1c), a solution of sulfamide (85 mg, 0.88 mmol) in THF (2 ml) was added rapidly. After 3.5h, water (5 ml) followed by diethyl ether (10 ml) were added. The aqueous layer was separated and extracted further with diethly ether (3 x 5 ml). The organic extracts were combined and dried (MgSO4). Filtration and evaporation of filtrate gave a dark green glassy solid which was purified by column chromatography on silica using ethyl acetate-hexanes (25:75) as eluent. Pooling and evaporation of appropriate fractions gave a pale yellow powder (178.9 mg, 32%). M.p. 183-184 °C (decomposed); IR (KBr) νₘₐₓ 3035, 3216, 2927, 2875, 1458, 1381, 1348, 1167, 1149, 1130, 1113, 1024, 916, 876, 735; ¹H-NMR: δ_{H} 5.42 (2H, broad-doublet, 2 x NH, *J* = 11.2 Hz), 5.35 (2H, s, 2 x H-12), 4.81 (2H, pseudo-triplet, 2 x H-10, *J* = 10.4 Hz), 2.36-2.29 (4H, m), 2.03-2.00 (2H, m), 1:89-1.86 (2H, m), 1.76-1.70 (4H, m), 1.57-1.54 (2H, m), 1.45 (6H, s, 2 x 3-Me), 1.44-1.42 (2H, m), 1.38-1.77 (6H, m), 1.02-0.97 (2H, m), 0.95(6H, d, 2 x 9-Me, *J* = 6.4 Hz), 0.91 (6H, d, 2 x 6-Me, *J* = 6.8 Hz); ¹³C-NMR: δ_{c} 104.32, 91.06, 82.80, 79.59, 51.68, 45.67, 37.30, 36.31, 34.09, 32.46, 25.71, 24.80, 21.55, 20.28, 13.42.

## Claims

1. A compound of the general formula I 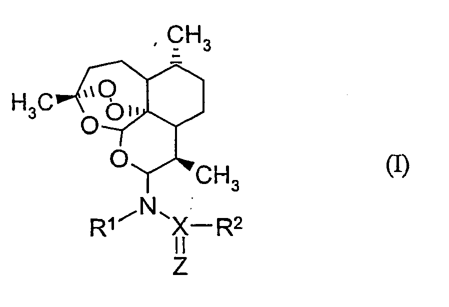 or a salt thereof, or a solvate thereof, or a solvate of a salt thereof,
in which
R¹ represents a hydrogen atom or an optionally substituted alkyl, alkenyl, alkynyl, cycloalkyl, aryl or aralkyl group;
X represents a carbon atom, a sulfur atom, a sulfoxide group S=O or a group PR³, P-O-R³ or P-N(R⁴)-R³ where R³ and R⁴ each independently represent a hydrogen atom or an optionally substituted alkyl, alkenyl, alkynyl, cycloakyl, aryl or aralkyl group;
Z represents an oxygen atom, a sulfur atom or a group NR⁵ where R⁵ represents a hydrogen atom or an optionally substituted alkyl, alkenyl, alkynyl, cycloalkyl, aryl or aralkyl group; and
R² represents a hydrogen atom or an optionally substituted alkyl, alkenyl, alkynyl, cycloalkyl, aryl or aralkyl group, or a group N(R⁶)₂, NHNH₂, NR⁶NHR⁶ or NR⁶N(R⁶)₂, or a group OR⁶ or SR⁶ where each R⁶ independently represents a hydrogen atom or an optionally substituted alkyl, alkenyl, alkynyl, cycloalkyl, aryl or aralkyl group, or a 10α-dihydroartemisinyl group, or R² represents a group OR⁷ or NR⁶R⁷ where R⁶ represents a group as defined above and R⁷ represents a bond attached as a substituent to R⁵ together with the interjacent group -X=Z- forming an optionally substituted heterocyclic group where Z represents a group NR⁵, or R⁷ represents a bond attached as a substituent to R¹ together with the interjacent group -N-X(=Z)- forming an optionally substituted heterocyclic group.

2. A compound according to claim 1 in which R¹ represents a hydrogen atom, a methyl group, ethyl group or longer chain alkyl group or a branched alkyl group containing up to 9 carbon atoms, preferably a hydrogen atom, a methyl group or an ethyl group.

3. A compound according to claim 1 or 2 in which X represents a carbon atom, a sulfur atom, or a group PR³, P-O-R³ or P-N(R⁴)-R³ where R³ and R⁴ each independently represent a C₆₋₁₈ aryl group or a 5- to 10-membered C-linked heteroaryl group or a 5- to 10-membered heterocyclyl-C₁₋₆ alkyl group optionally substituted by one or more substituents selected from the group consisting of halogen atoms, hydroxyl, C₁₋₄ alkyl, C₂₋₄ alkenyl, C₁₋₄ haloalkyl, C₁₋₄ alkoxy, C₁₋₄ haloalkoxy, amino, C₁₋₄ alkylamino, di(C₁₋₄ alkyl)amino and carboxyl groups.

4. A compound according to any of claims 1 to 3 in which Z represents an oxygen atom, or a group NR⁵ where R⁵ represents a hydrogen atom, a methyl group, ethyl group or longer chain alkyl group or branched alkyl group containing up to 9 carbon atoms, or a C₆₋₁₈ aryl group or a 5- to 10-membered C-linked heteroaryl group or a 5- to 10-membered heterocyclyl-C₁₋₆ alkyl group optionally substituted by one or more substituents selected from the group consisting of halogen atoms, hydroxyl, C₁₋₄ alkyl, C₂₋₄ alkenyl, C₁₋₄ haloalkyl, C₁₋₄ alkoxy, C₁₋₄ haloalkoxy, amino, C₁₋₄ alkylamino, di(C₁₋₄ alkyl)amino and carboxyl groups.

5. A compound according to any of the preceding claims in which R² represents a hydrogen atom or an optionally substituted alkyl, alkenyl, alkynyl, cycloalkyl, aryl or aralkyl group, or a group OR⁶, SR⁶, NH₂, NHR⁶, or N(R⁶)₂ where each R⁶ indepently represents a methyl group, ethyl group or longer chain alkyl group or branched alkyl group containing up to 9 carbon atoms atoms, or is a C₆₋₁₈ aryl group or a 5- to 10-membered C-linked heteroaryl group or a 5- to 10-membered heterocyclyl-C₁₋₆ alkyl group optionally substituted by one or more substituents selected from the group consisting of halogen atoms, hydroxyl, C₁₋₄ alkyl, C₂₋₄ alkenyl, C₁₋₄ haloalkyl, C₁₋₄ alkoxy; C₁₋₄, haloalkoxy, amino, C₁₋₄ alkylamino, di(C₁₋₄ alkyl)amino and carboxyl groups.

6. A compound according to any of the preceding claims in which R¹ represents a hydrogen atom or an optionally substituted alkyl, alkenyl, alkynyl, cycloalkyl, aryl or aralkyl group, preferably a hydrogen atom or an alkyl group, more preferably a hydrogen atom or a methyl group or an ethyl group; X represents a carbon, phosphorus or sulfur atom, preferably a carbon or sulfur atom; Z represents an oxygen atom or a group NR⁵ in where R⁵ represents a hydrogen atom or an optionally substituted alkyl, alkenyl, alkynyl, cycloalkyl, aryl or aralkyl group, preferably an oxygen atom; and R² represents a group OR⁶, SR⁶, NH₂, NHR⁶, or N(R⁶)₂ where each R⁶ independently represents a hydrogen atom or an optionally substituted alkyl, alkenyl, alkynyl, cycloalkyl, aryl or aralkyl group, or a 10α-dihydroartemisinyl group, preferably a hydrogen atom or an optionally substituted alkyl or aryl group, more preferably R² represents a group NH₂, or a group NHR⁶ where R⁶ represents an alkyl group, or a group N(R⁶)₂ where R⁶ represent identical or differentiated alkyl groups.

7. A compound according to any of the preceding claims in which R¹ represents a hydrogen atom, X represents a sulfoxide group S=O, Z represents an oxygen atom, and R² represents a group NH₂; or in which R¹ represents a hydrogen atom, X represents a carbon atom, Z represents a group NH, and R² represents a group NHR⁶ where R⁶ represents a hydrogen atom or an optionally substituted alkyl, cycloalkyl, aryl or aralkyl group; or in which R¹ represents a hydrogen atom, X represents a carbon atom, Z represents an oxygen atom, and R² represents a group NHR⁶ where R⁶ is a hydrogen atom or an optionally substituted alkyl, cycloalkyl, aryl or aralkyl group.

8. A process for the preparation of a compound of the general formula I according to any of the preceding claims which comprises reacting a compound of the general formula II 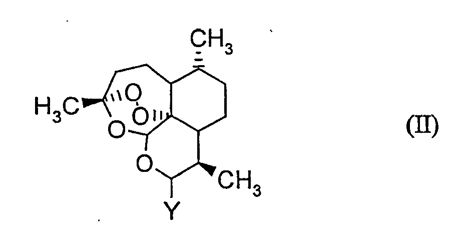 in which Y represents a group containing an oxygen atom attached to the carbon atom of the artemisinin nucleus and also to a hydrogen atom or trimethylsilyl group, with a suitable halogenating agent to form a compound of the general formula II in which Y represents a halogen atom; and, if desired, reacting the compound of general formula II thus formed with an amine of the general formula R¹NHX(=Z)R² where R¹, R², X and Z are as defined any of the preceding claims to form a compound of general formula I.

9. A compound according to any of claims 1 to 7 for use in the treatment and/or prophylaxis of a disease.

10. A pharmaceutical composition which comprises a carrier and, as active ingredient, a compound according to any of claims 1 to 7.

11. Use of a compound according to any of claims 1 to 7 for the manufacture of a medicament for the treatment and/or prophylaxis of a disease caused by infection with a parasite.

12. A pharmaceutical composition according to claim 10 for the treatment and/or prophylaxis of a disease caused by infection with a parasite.

## Patentansprüche

1. Verbindung der allgemeinen Formel I 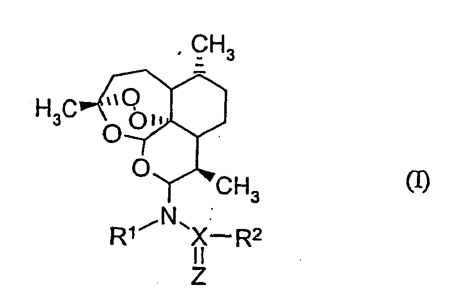 oder ein Salz davon, ein Solvat davon oder ein Solvat eines Salzes davon,
worin:
R¹ für ein Wasserstoffatom oder eine gegebenenfalls substituierte Alkyl-, Alkenyl-, Alkinyl-, Cycloalkyl-, Aryl- oder Aralkylgruppe steht;
X für ein Kohlenstoffatom, ein Schwefelatom, eine Sulfoxidgruppe S=O oder eine Gruppe PR³, P-O-R³ oder P-N(R⁴)-R³ steht, worin R³ und R⁴ jeweils unabhängig voneinander für ein Wasserstoffatom oder eine gegebenenfalls substituierte Alkyl-, Alkenyl-, Alkinyl-, Cycloalkyl-, Aryl- oder Aralkylgruppe stehen;
Z für ein Sauerstoffatom, ein Schwefelatom oder eine Gruppe NR⁵ steht, worin R⁵ für ein Wasserstoffatom oder eine gegebenenfalls substituierte Alkyl-, Alkenyl-, Alkinyl-, Cycloalkyl-, Aryl- oder Aralkylgruppe steht; und
R² für ein Wasserstoffatom oder eine gegebenenfalls substituierte Alkyl-, Alkenyl-, Alkinyl-, Cycloalkyl-, Aryl- oder Aralkylgruppe oder eine Gruppe N(R⁶)₂, NHNH₂, NR ⁶NHR⁶ oder NR⁶N(R⁶)₂ oder eine Gruppe OR⁶ oder SR⁶ steht, worin die R⁶ jeweils unabhängig voneinander für ein Wasserstoffatom oder eine gegebenenfalls substituierte Alkyl-, Alkenyl-, Alkinyl-, Cycloalkyl-, Aryl- oder Aralkylgruppe oder eine 10α-Dihydroartemisinylgruppe steht oder R² für eine Gruppe OR⁷ oder NR⁶R⁷ steht, worin R⁶ für eine zuvor definierte Gruppe steht und R⁷ für eine Bindung steht, die als Substituent an R⁵ gebunden ist und zusammen mit der Zwischengruppe -X=Z- eine gegebenenfalls substituierte heterozyklische Gruppe bildet, wenn Z für eine Gruppe NR⁵ steht, oder R⁷ für eine Bindung steht, die als Substituent an R¹ gebunden ist und zusammen mit der Zwischengruppe -N-X(=Z)- eine gegebenenfalls substituierte heterozyklische Gruppe bildet.

2. Verbindung nach Anspruch 1, in der R¹ für ein Wasserstoffatom, eine Methylgruppe, Ethylgruppe oder eine längerkettige Alkylgruppe oder eine verzweigte Alkylgruppe, die bis zu 9 Kohlenstoffatomen enthält, vorzugsweise für ein Wasserstoffatom, eine Methylgruppe oder eine Ethylgruppe, steht.

3. Verbindung nach Anspruch 1 oder 2, in der X für ein Kohlenstoffatom, ein Schwefelatom oder eine Gruppe PR³, P-O-R³ oder P-N(R⁴)-R³ steht, worin R³ und R⁴ jeweils unabhängig voneinander für eine C₆₋₁₈-Arylgruppe oder eine 5- bis 10-gliedrige, über C verbundene Heteroarylgruppe oder eine C₁₋₆-Alkylgruppe mit einem 5- bis 10-gliedrigen heterozyklischen Substituenten, die gegebenenfalls mit einem oder mehreren Substituenten, ausgewählt aus der aus Halogenatomen, Hydroxyl-, C₁₋₄₋Alkyl-, C₂₋₄-Alkenyl-, C₁₋₄-Halogenalkyl-, C₁₋₄-Alkoxy-, C₁₋₄-Halogenalkoxy-, Amino-, C₁₋₄-Alkylamino-, Di(C₁₋₄-alkyl)amino- und Carboxylgruppen bestehenden Gruppe, substituiert ist, stehen.

4. Verbindung nach einem der Ansprüche 1 bis 3, in der Z für ein Sauerstoffatom oder eine Gruppe NR⁵ steht, worin R⁵ für ein Wasserstoffatom, eine Methylgruppe, Ethylgruppe oder eine längerkettige Alkylgruppe oder eine verzweigte Alkylgruppe mit bis zu 9 Kohlenstoffatomen oder eine C₆₋₁₈-Arylgruppe oder eine 5- bis 10-gliedrige, über C verbundene Heteroarylgruppe oder eine C₁₋₆-Alkylgruppe mit einem 5- bis 10-gliedrigen heterozyklischen Substituenten, die gegebenenfalls mit einem oder mehreren Substituenten, ausgewählt aus der aus Halogenatomen, Hydroxyl-, C₁₋₄₋Alkyl-, C₂₋₄-Alkenyl-, C₁₋₄-Halogenalkyl-, C₁₋₄-Alkoxy-, C₁₋₄-Halogenalkoxy-, Amino-, C₁₋₄-Alkylamino-, Di(C₁₋₄-alkyl)amino- und Carboxylgruppen bestehenden Gruppe, substituiert ist, steht.

5. Verbindung nach einem der vorangehenden Ansprüche, in der R² für ein Wasserstoffatom oder eine gegebenenfalls substituierte Alkyl-, Alkenyl-, Alkinyl-, Cycloalkyl-, Aryl- oder Aralkylgruppe oder eine Gruppe OR⁶, SR⁶, NH₂, NHR⁶ oder N(R⁶)₂ steht, worin die R⁶ jeweils unabhängig voneinander für eine Methylgruppe, Ethylgruppe oder eine längerkettige Alkylgruppe oder eine verzweigte Alkylgruppe mit bis zu 9 Kohlenstoffatomen oder eine C₆₋₁₈-Arylgruppe oder eine 5- bis 10-gliedrige, über C verbundene Heteroarylgruppe oder eine C₁₋₆-Alkylgruppe mit einem 5- bis 10-gliedrigen heterozyklischen Substituenten, die gegebenenfalls mit einem oder mehreren Substituenten, ausgewählt aus der aus Halogenatomen, Hydroxyl-, C₁₋₄-Alkyl-, C₂₋₄₋Alkenyl-, C₁₋₄-Halogenalkyl-, C₁₋₄-Alkoxy-, C₁₋₄-Halogenalkoxy-, Amino-, C₁₋₄-Alkylamino-, Di(C₁₋₄-alkyl)amino- und Carboxylgruppen bestehenden Gruppe, substituiert ist, steht.

6. Verbindung nach einem der vorangehenden Ansprüche, in der R¹ für ein Wasserstoffatom oder eine gegebenenfalls substituierte Alkyl-, Alkenyl-, Alkinyl-, Cycloalkyl-, Aryl- oder Aralkylgruppe, vorzugsweise ein Wasserstoffatom oder eine Alkylgruppe, noch bevorzugter ein Wasserstoffatom oder eine Methylgruppe oder eine Ethylgruppe, steht; X für ein Kohlenstoff-, Phosphor- oder Schwefelatom, vorzugsweise ein Kohlenstoff- oder Schwefelatom, steht; Z für ein Sauerstoffatom oder eine Gruppe NR⁵, worin R⁵ für ein Wasserstoffatom oder eine gegebenenfalls substituierte Alkyl-, Alkenyl-, Alkinyl-, Cycloalkyl-, Aryl- oder Aralkylgruppe steht, vorzugsweise für ein Sauerstoffatom, steht; und R² für eine Gruppe OR⁶, SR⁶, NH₂, NHR⁶ oder N(R⁶)₂ steht, worin die R⁶ jeweils unabhängig voneinander für ein Wasserstoffatom oder eine gegebenenfalls substituierte Alkyl-, Alkenyl-, Alkinyl-, Cycloalkyl-, Aryl- oder Aralkylgruppe oder eine 10α-Dihydroartemisinylgruppe, vorzugsweise ein Wasserstoffatom oder eine gegebenenfalls substituierte Alkyl- oder Arylgruppe steht, noch bevorzugter R² für eine Gruppe NH₂ oder eine Gruppe NHR⁶, worin R⁶ für eine Alkylgruppe steht, oder eine Gruppe N(R ⁶)₂ steht, worin R⁶ für gleiche oder unterschiedliche Alkylgruppen steht.

7. Verbindung nach einem der vorangehenden Ansprüche, in der R¹ für ein Wasserstoffatom steht, X für eine Sulfoxidgruppe S=O steht, Z für ein Sauerstoffatom steht und R² für eine Gruppe NH₂ steht; oder in der R¹ für ein Wasserstoffatom steht, X für ein Kohlenstoffatom steht, Z für eine Gruppe NH steht und R² für eine Gruppe NHR⁶ steht, worin R⁶ für ein Wasserstoffatom oder eine gegebenenfalls substituierte Alkyl-, Cycloalkyl-, Aryl- oder Aralkylgruppe steht; oder in der R¹ für ein Wasserstoffatom steht, X für ein Kohlenstoffatom steht, Z für ein Sauerstoffatom steht und R² für eine Gruppe NHR⁶ steht, worin R⁶ ein Wasserstoffatom oder eine gegebenenfalls substituierte Alkyl-, Cycloalkyl-, Aryl- oder Aralkylgruppe ist.

8. Verfahren zur Herstellung einer Verbindung der allgemeinen Formel I nach einem der vorangehenden Ansprüche, umfassend das Umsetzen einer Verbindung der allgemeinen Formel II 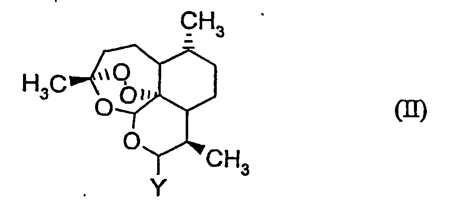 in der Y für eine Gruppe steht, die ein Sauerstoffatom enthält, das an das Kohlenstoffatom des Artemisininkerns und auch an ein Wasserstoffatom oder eine Trimethylsilylgruppe gebunden ist, mit einem geeigneten Halogenierungsmittel, um eine Verbindung der allgemeinen Formel 11 zu bilden, in der Y für ein Halogenatom steht; sowie gegebenenfalls das Umsetzen der so gebildeten Verbindung der allgemeinen Formel 11 mit einem Amin der allgemeinen Formel R¹NHX(=Z)R², worin R¹, R², X und Z wie in den vorangehenden Ansprüchen definiert sind, um eine Verbindung der allgemeinen Formel I zu bilden.

9. Verbindung nach einem der Ansprüche 1 bis 7 zur Verwendung bei der Behandlung einer und/oder Prophylaxe gegen eine Erkrankung.

10. Pharmazeutische Zusammensetzung, die einen Träger und als Wirkstoff eine Verbindung nach einem der Ansprüche 1 bis 7 umfasst.

11. Verwendung einer Verbindung nach einem der Ansprüche 1 bis 7 zur Herstellung eines Medikaments zur Behandlung einer und/oder Prophylaxe gegen eine Erkrankung, die durch Infektion mit einem Parasiten hervorgerufen wird.

12. Pharmazeutische Zusammensetzung nach Anspruch 10 zur Behandlung einer und/oder Prophylaxe gegen eine Erkrankung, die durch Infektion mit einem Parasiten hervorgerufen wird.

## Revendications

1. Composé de formule générale 1 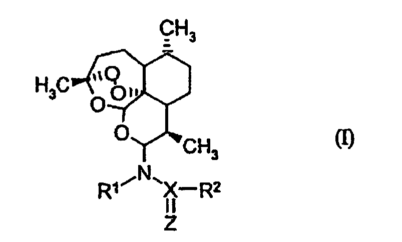 ou un de ses sels, ou un de ses produits de solvatation, ou un produit de solvatation d'un de ses sels,
dans laquelle
R¹ représente un atome d'hydrogène ou un groupe alkyle, alcényle, alcynyle, cycloalkyle, aryle ou aralkyle facultativement substitué ;
X représente un atome de carbone, un atome de soufre, un groupe sulfoxyde S=O ou un groupe PR³, P-O-R³ ou P-N (R⁴) -R³ où R³ et R⁴ représentent chacun indépendamment un atome d'hydrogène ou un groupe alkyle, alcényle, alcynyle, cycloalkyle, aryle ou aralkyle facultativement substitué ;
Z représente un atome d'oxygène, un atome de soufre ou un groupe NR⁵ où R⁵ représente un atome d'hydrogène ou un groupe alkyle, alcényle, alcynyle, cycloalkyle, aryle ou aralkyle facultativement substitué ; et
R² représente un atome d'hydrogène ou un groupe alkyle, alcényle, alcynyle, cycloalkyle, aryle ou aralkyle facultativement substitué, ou un groupe N(R⁶)₂, NHNH₂, NR⁶NHR⁶ ou NR⁶N(R⁶)₂, ou un groupe OR⁶ ou SR⁶ où chaque groupe R⁶ représente indépendamment un atome d'hydrogène ou un groupe alkyle, alcényle, alcynyle, cycloalkyle, aryle ou aralkyle facultativement substitué, ou un groupe 10α-dihydroartémisinyle, ou bien R² représente un groupe OR⁷ ou NR⁶R⁷ où R⁶ représente un groupe répondant à la définition précitée et R⁷ représente une liaison fixée comme substituant à R⁵ conjointement avec le groupe intermédiaire -X=Z- en formant un groupe hétérocyclique facultativement substitué où Z représente un groupe NR⁵, ou bien R⁷ représente une liaison fixée comme substituant à R¹ conjointement avec le groupe intermédiaire -N-X(=Z)- en formant un groupe hétérocyclique facultativement substitué.

2. Composé suivant la revendication 1, dans lequel R¹ représente un atome d'hydrogène, un groupe méthyle, un groupe éthyle ou un groupe alkyle à chaîne plus longue ou un groupe alkyle ramifié contenant jusqu'à 9 atomes de carbone, de préférence un atome d'hydrogène, un groupe méthyle ou un groupe éthyle.

3. Composé suivant la revendication 1 ou 2, dans lequel X représente un atome de carbone, un atome de soufre ou un groupe PR³, P-O-R³ ou P-N (R⁴) -R³ où R³ et R⁴ représentent chacun indépendamment un groupe aryle en C₆ à C₁₈ ou un groupe hétéroaryle penta- à décagonal lié par C ou (hétérocyclyle penta- à décagonal)(alkyle en C₁ à C₆) facultativement substitué avec un ou plusieurs substituants choisis dans le groupe consistant en des atomes d'halogènes, des groupes hydroxyle, alkyle en C₁ à C₄, alcényle en C₂ à C₄, halogénalkyle en C₁ à C₄, alkoxy en C₁ à C₄, halogénalkoxy en C₁ à C₄, amino, alkylamino en C₁ à C₄, di (alkyle en C₁ à C₄)amino et carboxyle.

4. Composé suivant l'une quelconque des revendications 1 à 3, dans lequel Z représente un atome d'oxygène ou un groupe NR⁵ dans lequel R⁵ représente un atome d'hydrogène, un groupe méthyle, un groupe éthyle ou un groupe alkyle à chaîne longue ou un groupe alkyle ramifié contenant jusqu'à 9 atomes de carbone, ou un groupe aryle en C₆ à C₁₈ ou hétéroaryle penta- à décagonal lié par C ou (hétérocyclyle penta- à décagonal) (alkyle en C₁ à C₆) facultativement substitué avec un ou plusieurs substituants choisis dans le groupe consistant en des atomes d'halogènes, des groupes hydroxyle, alkyle en C₁ à C₄, alcényle en C₂ à C₄, halogénalkyle en C₁ à C₄, alkoxy en C₁ à C₄, halogénalkoxy en C₁ à C₄, amino, alkylamino en C₁ à C₄, di(alkyle en C₁ à C₄) amino et carboxyle.

5. Composé suivant l'une quelconque des revendications précédentes, dans lequel R² représente un atome d'hydrogène ou un groupe alkyle, alcényle, alcynyle, cycloalkyle, aryle ou aralkyle facultativement substitué, ou un groupe OR⁶, SR⁶, NH₂, NHR⁶ ou N(R⁶)₂ dans lequel chaque groupe R⁶ représente indépendamment un groupe méthyle, un groupe éthyle ou un groupe alkyle à chaîne plus longue ou un groupe alkyle ramifié contenant jusqu'à 9 atomes de carbone, ou représente un groupe aryle en C₆ à C₁₈ ou un groupe hétéroaryle penta- à décagonal lié par C ou un groupe (hétérocyclyle penta- à décagonal)(alkyle en C₁ à C₆) facultativement substitué avec un ou plusieurs substituants choisis dans le groupe consistant en des atomes d'halogènes, des groupes hydroxyle, alkyle en C₁ à C₄, alcényle en C₂ à C₄, halogénalkyle en C₁ à C₄, alkoxy en C₁ à C₄, halogénalkoxy en C₁ à C₄, amino, alkylamino en C₁ à C₄, di (alkyle en C₁à C₄)amino et carboxyle.

6. Composé suivant l'une quelconque des revendications précédentes, dans lequel R¹ représente un atome d'hydrogène ou un groupe alkyle, alcényle, alcynyle, cycloalkyle, aryle ou aralkyle facultativement substitué, avantageusement un atome d'hydrogène ou un groupe alkyle, plus avantageusement un atome d'hydrogène ou un groupe méthyle ou un groupe éthyle ; X représente un atome de carbone, de phosphore ou de soufre, de préférence un atome de carbone ou de soufre ; Z représente un atome d'oxygène ou un groupe NR⁵ dans lequel R⁵ représente un atome d'hydrogène ou un groupe alkyle, alcényle, alcynyle, cycloalkyle, aryle ou aralkyle facultativement substitué, de préférence un atome d'oxygène ; et R²représente un groupe OR⁶, SR⁶, NH₂, NHR⁶ ou N (R⁶) ₂ dans lequel chaque groupe R⁶ représente indépendamment un atome d'hydrogène ou un groupe alkyle, alcényle, alcynyle, cycloalkyle, aryle ou aralkyle facultativement substitué, ou un groupe 10α-dihydroartémisinyle, de préférence un atome d'hydrogène ou un groupe alkyle ou aryle facultativement substitué ; plus avantageusement, R² représente un groupe NH₂, ou un groupe NHR⁶ dans lequel R⁶ représente un groupe alkyle, ou un groupe N(R⁶)₂ dans lequel R⁶ représente des groupes alkyle identiques ou différents.

7. Composé suivant l'une quelconque des revendications précédentes, dans lequel R¹ représente un atome d'hydrogène, X représente un groupe sulfoxyde S=O, Z représente un atome d'oxygène et R² représente un groupe NH₂ ; ou dans lequel R¹ représente un atome d'hydrogène, X représente un atome de carbone, Z représente un groupe NH et R² représente un groupe NHR⁶ dans lequel R⁶ représente un atome d'hydrogène ou un groupe alkyle, cycloalkyle, aryle ou aralkyle facultativement substitué ; ou dans lequel R¹ représente un atome d'hydrogène, X représente un atome de carbone, Z représente un atome d'oxygène et R² représente un groupe NHR⁶ dans lequel R⁶ représente un atome d'hydrogène ou un groupe alkyle, cycloalkyle, aryle ou aralkyle facultativement substitué.

8. Procédé pour la préparation d'un composé de formule générale I suivant l'une quelconque des revendications précédentes, qui comprend la réaction d'un composé de formule générale II 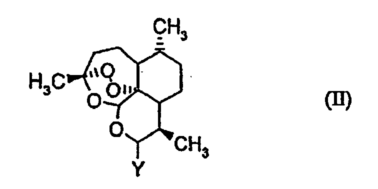 dans laquelle Y représente un groupe contenant un atome d'oxygène fixé à l'atome de carbone du noyau artémisinine et aussi un atome d'hydrogène ou un groupe triméthylsilyle, avec un agent d'halogénation convenable pour former un composé de formule générale II dans laquelle Y représente un atome d'halogène ; et, si désiré, la réaction du composé de formule générale II ainsi formé avec une amine de formule générale R¹NHX (=Z) R² dans laquelle R¹, R², X et Z répondent aux définitions de l'une quelconque des revendications précédentes, pour former un composé de formule générale I.

9. Composé suivant l'une quelconque des revendications 1 à 7, pour une utilisation dans le traitement et/ou la prophylaxie d'une maladie.

10. Composition pharmaceutique qui comprend un support et, comme ingrédient actif, un composé suivant l'une quelconque des revendications 1 à 7.

11. Utilisation d'un composé suivant l'une quelconque des revendications 1 à 7 pour la production d'un médicament pour le traitement et/ou la prophylaxie d'une maladie provoquée par une infection par un parasite.

12. Composition pharmaceutique suivant la revendication 10, pour le traitement et/ou la prophylaxie d'une maladie provoquée par l'infection par un parasite.
